(19) **Europäisches Patentamt / European Patent Office / Office européen des brevets**

(11) **EP 3 266 521 A1**

(12) **EUROPEAN PATENT APPLICATION**
published in accordance with Art. 153(4) EPC

(43) Date of publication:
**10.01.2018 Bulletin 2018/02**

(21) Application number: **16758958.9**

(22) Date of filing: **02.03.2016**

(51) Int Cl.:
**B01J 23/887** (2006.01)   **B01J 35/08** (2006.01)
**B01J 37/02** (2006.01)   **B01J 37/08** (2006.01)
**C07C 5/48** (2006.01)   **C07C 11/167** (2006.01)
**C07B 61/00** (2006.01)

(86) International application number:
**PCT/JP2016/056416**

(87) International publication number:
**WO 2016/140265 (09.09.2016 Gazette 2016/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **03.03.2015   JP 2015041831**
**03.03.2015   JP 2015041832**
**03.03.2015   JP 2015041833**

(71) Applicant: **Nippon Kayaku Kabushiki Kaisha Tokyo 100-0005 (JP)**

(72) Inventors:
• **OKUMURA Shigeki**
  **Sanyoonoda-shi**
  **Yamaguchi 757-0002 (JP)**

• **NAKAZAWA Yuuta**
  **Sanyoonoda-shi**
  **Yamaguchi 757-0002 (JP)**
• **MOTOMURA Hiroki**
  **Sanyoonoda-shi**
  **Yamaguchi 757-0002 (JP)**
• **NISHIZAWA Bungo**
  **Sanyoonoda-shi**
  **Yamaguchi 757-0002 (JP)**
• **NAKAYAMA Koji**
  **Sanyoonoda-shi**
  **Yamaguchi 757-0002 (JP)**

(74) Representative: **Gille Hrabal**
  **Brucknerstrasse 20**
  **40593 Düsseldorf (DE)**

(54) **CATALYST FOR CONJUGATED DIOLEFIN PRODUCTION AND METHOD FOR PRODUCING SAME**

(57)     Provided are a catalyst that may suppress the production of a coke-like substance in a reaction for producing a conjugated diolefin from a mixed gas including a monoolefin having 4 or more carbon atoms and molecular oxygen, by catalytic oxidative dehydrogenation; and a method for producing the catalyst.

A composite metal oxide catalyst for producing a conjugated diolefin from a mixed gas including a monoolefin having 4 or more carbon atoms and molecular oxygen by a catalytic oxidative dehydrogenation reaction, the composite metal oxide catalyst having a relative intensity range of X-ray diffraction peaks represented by the following Formula (A): $0 \leq$ Rh (= Ph1/Ph2) < 0.8 (A) wherein Ph1 represents the maximum peak height within the range of $2\theta = 28.1° \pm 0.2°$ for the X-ray diffraction peaks; Ph2 represents the maximum peak height within the range of $2\theta = 27.9° \pm 0.2°$ for the X-ray diffraction peaks; and Rh represents the relative intensity ratio of Ph1 with respect to Ph2.

EP 3 266 521 A1

**Description**

Technical Field

The present invention relates to a composite metal oxide catalyst (hereinafter, also described as catalyst) that produces a conjugated diolefin from a mixed gas including a monoolefin having 4 or more carbon atoms and molecular oxygen by a catalytic oxidative dehydrogenation reaction, and a method for producing the same.

Background Art

Butadiene, which is a raw material of synthetic rubber or the like, has been conventionally produced industrially by thermal decomposition of a naphtha fraction and extraction; however, since there are concerns about deterioration of stable supply to the market in the future, there is a demand for a new method for producing butadiene. Thus, attention has been paid to a method of oxidatively dehydrogenating n-butene from a mixed gas including n-butene and molecular oxygen in the presence of a catalyst. However, there are concerns that as a coke-like substance originating from a reaction product and/or reaction byproducts is precipitated in or adheres to the catalyst surface, inert materials, the interior of a reaction tube, or the interior of the subsequent process facilities, various problems such as inhibition of the circulation of the reaction gas in an industrial plant, blocking of the reaction tube, shutdown of the plant resulting therefrom, and a decrease in the yield, may be brought about. For the purpose of avoiding the problems described above, in an industrial plant, generally, the reaction is stopped before blocking occurs, and a regeneration treatment of combusting and removing the coke-like substance by means of temperature increase of the reaction bath temperature or the like is carried out. Since stopping a reaction that is in steady operation and then performing a regeneration treatment leads to the deterioration of economic efficiency in an industrial plant, it is preferable that the production of the coke-like substance is suppressed as far as possible. Thus, it is known to make improvements in the catalyst composition, reaction conditions and the like as described below, for the purpose of suppressing the generation of the coke-like substance.
Regarding Patent Literatures related to the catalyst composition, Patent Literature 1 discloses a catalyst composition that suppresses side-production of a coke-like substance and enables stable continuation of the reaction, and Patent Literature 3 discloses a catalyst composition and the acidity of the catalyst that give high conjugated diolefin yield. Furthermore, the X-ray diffraction peak ratio of a catalyst that may suppress the generation of a coke-like substance is defined in Patent Literature 4. Regarding Patent Literatures related to the reaction conditions, reaction conditions that prevent accumulation of a coke-like substance on a catalyst are defined in Patent Literature 2. In regard to all of these Patent Literatures, when the economic efficiency in the industrial plant, that is, the amount of precipitation of a coke-like substance with respect to the duration of the reaction, the frequency of regeneration, and the like are taken into consideration, it is inappropriate to say that the production of coke-like substances has been sufficiently suppressed. Thus, it has been desired that a catalyst composition that may further suppress the production of a coke-like substance and improve the long-term stability of the reaction, and reaction conditions for the catalyst, will be suggested.
From the viewpoint of the economic efficiency in an industrial plant, it is also definitely important that butadiene, which is a target product, is obtained with high yield. A low butadiene yield means that the yield of byproducts is relatively high; however, in this case, in order to obtain butadiene of high purity as a final product in an industrial plant, a purification system with superior performance is needed, and there are concerns about the facilities cost of such a system being increased.
Furthermore, from a different viewpoint on the long-term stability of the reaction in an industrial plant, it is also important that the mechanical strength of the catalyst itself is high. If the catalyst has insufficient mechanical strength, problems such as damage of the catalyst caused by the production of the coke-like substance, and/or damage of the catalyst caused by combustion gas in the regeneration treatment, and/or degradation of the catalyst, may be considered. Furthermore, there are concerns that these problems may lead to other problems such as accumulation of damaged catalyst in the reactor, an increase in the pressure loss, undesirable reactions caused by the catalyst that has locally accumulated in the reactor, and incorporation of the accumulated catalyst into the purification system in the subsequent stages.

Citation List

Patent Literature

Patent Literature 1: JP 2013-100244 A
Patent Literature 2: JP 2011-148765 A

Patent Literature 3: JP 2013-146655 A
Patent Literature 4: JP 2013-202459 A

Non Patent Literature

Non-Patent Literature 1: M.E. Levin, et al. (2004) J. Haz. Mat., 115, 71-90
Non-Patent Literature 2: W. Martir, et al. (1981) J. Am. Chem. Soc., 103, 3728-3732
Non-Patent Literature 3: J.H. Lunsford (1989) Langmuir, 5, 12-16
Non-Patent Literature 4: M.T. Le, et al. (2005) App. Cat. A, 282, 189-194

Summary

Technical Problem

An object of the present invention is to provide a catalyst that may suppress the production of a coke-like substance and may improve the long-term stability of the reaction, in a reaction for producing a conjugated diolefin from a mixed gas including a monoolefin having 4 or more carbon atoms and molecular oxygen by catalytic oxidative dehydrogenation, and to provide a method for producing the same.

Solution to Problem

The inventors of the present invention conducted a thorough investigation in order to solve the problems described above, and as a result, the inventors found that when the relative intensity ratio of a particular crystal phase in relation to the X-ray diffraction peaks of a composite metal oxide catalyst satisfies a particular range, production of the coke-like substance may be significantly suppressed, and the problems described above may be solved. Thus, the inventors completed the present invention.

The present invention has the features of the following items (1) to (10) singly or in combination. That is, the present invention may include the following embodiments.

The present invention relates to

(1) a composite metal oxide catalyst for producing a conjugated diolefin from a mixed gas including a monoolefin having 4 or more carbon atoms and molecular oxygen by a catalytic oxidative dehydrogenation reaction, the composite metal oxide catalyst having a relative intensity range of X-ray diffraction peaks represented by the following Formula (A):

$$0 \leq Rh \; (= Ph1/Ph2) < 0.8 \qquad \qquad (A)$$

wherein Ph1 represents the maximum peak height within the range of 2θ = 28.1° ± 0.2° for the X-ray diffraction peaks; Ph2 represents the maximum peak height within the range of 2θ = 27.9° ± 0.2° for the X-ray diffraction peaks; and Rh represents the relative intensity ratio of Ph1 with respect to Ph2,

(2) a composite metal oxide catalyst for producing a conjugated diolefin from a mixed gas including a monoolefin having 4 or more carbon atoms and molecular oxygen by a catalytic oxidative dehydrogenation reaction, the composite metal oxide catalyst having a relative area intensity range of X-ray diffraction peaks represented by the following Formula (B):

$$0 \leq Ra \; (= Pa1/Pa2) < 0.5 \qquad \qquad (B)$$

wherein Pa1 represents the peak area obtainable by fitting within the range of 2θ = 28.1° ± 0.2° for the X-ray diffraction peaks; Pa2 represents the peak area obtainable by fitting within the range of 2θ = 27.9° for the X-ray diffraction peaks; and Ra represents the relative area intensity ratio of Pa1 with respect to Pa2,

(3) the composite metal oxide catalyst according to claim 1 or claim 2, wherein the composite metal oxide catalyst includes molybdenum (Mo), bismuth (Bi), an alkali metal (AM), and if necessary, an alkaline earth metal (AEM), and the molar ratio of each component satisfies the following Formula (C):

$$0.02 \leq Re < 0.80 \qquad\qquad (C)$$

wherein

Re = (Content (mol) of Bi)/{(content (mol) of Mo) − (content (mol) of metal components other than Mo and Bi)},

(4) the composite metal oxide catalyst according to any of (1) to (3), wherein the composite metal oxide catalyst satisfies the following composition formula:

$$Mo_{12}Bi_aFe_bCo_cNi_dX_eY_fZ_gO_h$$

wherein Mo represents molybdenum; Bi represents bismuth; Fe represents iron; Co represents cobalt; Ni represents nickel; X represents at least one alkali metal element selected from lithium, sodium, potassium, rubidium, and cesium; Y represents at least one alkaline earth metal element selected from magnesium, calcium, strontium, and barium; Z represents at least one element selected from lanthanum, cerium, praseodymium, neodymium, samarium, europium, antimony, tungsten, lead, zinc, cerium, and thallium; O represents oxygen; a, b, c, d, e, and f represent the atomic ratios of bismuth, iron, cobalt, nickel, the alkali metal, the alkaline earth metal, and Z, respectively, with respect to molybdenum 12, and in the ranges of $0.3 < a < 3.5$, $0.6 < b < 3.4$, $5 < c < 8$, $0 < d < 3$, $0 < e < 0.5$, $0 \leq f \leq 4.0$, and $0 \leq g \leq 2.0$; and h represents a value satisfying the oxidation states of other elements,

(5) a method for producing the composite metal oxide catalyst for conjugated diolefin production according to (3), the method including:

a step of preparing a slurry including a compound containing molybdenum; a compound containing bismuth; a compound containing at least one alkali metal; and if necessary, a compound containing at least one alkaline earth metal, and drying the slurry to obtain a dry powder; and
a step of calcining the dry powder at a temperature of from 200°C to 600°C,

(6) a method for producing the composite metal oxide catalyst for conjugated diolefin production according to (4), the method including:

a step of preparing a slurry including a compound containing molybdenum; a compound containing bismuth; a compound containing iron; a compound containing cobalt; a compound containing nickel; a compound containing at least one alkali metal element selected from lithium, sodium, potassium, rubidium, and cesium; a compound containing at least one alkaline earth metal element selected from magnesium, calcium, strontium, and barium; and a compound containing at least one element Z selected from lanthanum, cerium, praseodymium, neodymium, samarium, europium, antimony, tungsten, lead, zinc, cerium, and thallium,
provided that a, b, c, d, e, and f represent the atomic ratios of bismuth, iron, cobalt, nickel, the alkali metal, the alkaline earth metal, and Z, respectively, with respect to molybdenum 12, and in the ranges of $0.3 < a < 3.5$, $0.6 < b < 3.4$, $5 < c < 8$, $0 < d < 3$, $0 < e < 0.5$, $0 \leq f \leq 4.0$, $0 \leq g \leq 2.0$;
a step of drying the slurry to obtain a dry powder; and
a step of calcining the dry powder at a temperature of from 200°C to 600°C,

(7) a supported catalyst for conjugated diolefin production, the supported catalyst including the composite metal oxide catalyst according to any of (1) to (4) supported on a support,
(8) the supported catalyst according to (7), wherein the support is a spherical support,
(9) the supported catalyst according to (7) or (8), wherein the average particle size is from 3.0 mm to 10.0 mm, and the support ratio of the composite metal oxide catalyst is from 20% by weight to 80% by weight, and
(10) a method for producing the supported catalyst for conjugated diolefin production according to any of (7) to (9), the method including a molding step of coating the support with the composite metal oxide catalyst together with a binder.

Advantageous Effects of Invention

The present invention provides a catalyst which may suppress the production of a coke-like substance and may improve the long-term stability of the reaction, in a reaction for producing a conjugated diolefin from a mixed gas including a monoolefin having 4 or more carbon atoms and molecular oxygen by catalytic oxidative dehydrogenation, and also provides a method for producing the catalyst.

Description of Embodiments

The method for analyzing X-ray diffraction peaks according to the present invention will be explained below. The term X-ray diffraction peaks refers to a chart of scattering intensity with respect to a particular value of the X-ray diffraction angle $2\theta$, and regarding the analyzer, analysis conditions and the like for the X-ray diffraction peaks, the details do not matter as long as the analyzer and the analysis conditions are generally known.

Next, the method for calculating the relative intensity ratio Rh of X-ray diffraction peaks according to the present invention will be described.

The diffraction peak in the range of $2\theta = 27.9° \pm 0.2°$ in an X-ray diffraction analysis is designated as Peak 2, the diffraction peak in the range of $2\theta = 28.1° \pm 0.2°$ is designated as Peak 1, and while the respective values of $2\theta$ that give the maximum values in the respective ranges of $2\theta$ for the peaks are considered as peak centers, the heights of the peaks at the peak centers of Peak 2 and Peak 1 are designated as Ph2 and Ph1, respectively. The relative intensity ratio Rh is calculated from Ph1 and Ph2 thus obtained, by the following formula:

$$Rh \ (= Ph1/Ph2)$$

Next, the method for calculating the relative area intensity ratio Ra of the X-ray diffraction peaks according to the present invention will be described.

First, the peak area of Peak 1 and Peak 2 are needed, and as long as the method for calculating the peak area is a generally known method, the details do not matter.

A preferable example will be described below. The X-ray diffraction peaks thus obtained are inputted to a personal computer as electronic data. With respect to the respective peak centers of Peak 2 and Peak 1, the peak shape is assumed to be a split type pseudo-Voigt function, and fitting is performed under the condition that the asymmetric factor is fixed at 1. The peak areas of Peak 2 and Peak 1 obtained by the fitting are designated as Pa2 and Pa1, respectively, and the relative area intensity ratio Ra is calculated by the following formula:

$$Ra \ (= Pa1/Pa2)$$

The catalyst of the present invention is a composite metal oxide catalyst having a relative intensity ratio Rh of X-ray diffraction that is represented by the following Formula (A). Furthermore, Rh is preferably represented by the following Formula (A'), and most preferably represented by Formula (A'').

$$0 \leq Rh \ (= Ph1/Ph2) < 0.8 \tag{A}$$

$$0.08 \leq Rh \ (= Ph1/Ph2) < 0.8 \tag{A'}$$

$$0.12 \leq Rh \ (= Ph1/Ph2) < 0.75 \tag{A''}$$

The catalyst of the present invention is also a composite metal oxide catalyst whose relative area intensity ratio Ra of X-ray diffraction is represented by the following Formula (B). Furthermore, Ra is preferably represented by the following Formula (B'), and most preferably represented by Formula (B'').

$$0 \leq Ra \ (= Pa1/Pa2) < 0.5 \tag{B}$$

$$0.08 \leq Ra\ (= Pa1/Pa2) < 0.5 \qquad\qquad (B')$$

$$0.12 \leq Ra\ (= Pa1/Pa2) < 0.48 \qquad\qquad (B'')$$

According to the present invention, in regard to the question of why a composite metal oxide catalyst that satisfies the above-mentioned Formula (A) and/or Formula (B) may suppress the production of a coke-like substance in a reaction for producing a conjugated diolefin from a mixed gas including a monoolefin having 4 or more carbon atoms and molecular oxygen, the details are not clearly understood; however, the following may be considered as one of the causes. As a cause for the production of the coke-like substance, polymerization caused by a radical polymerization reaction that is characteristic of butadiene as disclosed in Non-Patent Literature 1 may be considered. On the other hand, in regard to bismuth molybdate that is formed from molybdenum and bismuth, which are known as catalytically active components of the present reaction, crystal phases include α-bismuth molybdate, β-bismuth molybdate, and γ-bismuth molybdate. Among these, γ-bismuth molybdate gives a larger amount of production of a radical active species compared to α-bismuth molybdate, and this is known from Non-Patent Literature 2 and Non-Patent Literature 3. That is, in regard to a composite metal oxide catalyst containing bismuth molybdate, it is speculated that if the content of γ-bismuth molybdate (corresponding to Peak 1) is small compared to the content of α-bismuth molybdate (corresponding to Peak 2), the radical polymerization reaction of butadiene is suppressed, and production of the coke-like substance is also suppressed. However, in regard to composite metal oxide catalysts obtained by adding a metal element other than molybdenum and bismuth as is the case of the present invention, it is not specifically known which peak of the X-ray diffraction pattern gives a relative intensity ratio in what range, in order to obtain an effect preferable for suppressing the production of the coke-like substance.

As explained above, in regard to a composite metal oxide catalyst obtained by adding a metal element other than molybdenum and bismuth, if it is wished to change the ratio of the contents of γ-bismuth molybdate and α-bismuth molybdate, the change may be achieved by arbitrary regulation of the composition ratio of each element of the composite metal oxide catalyst that will be described below and various production parameters for the catalyst production; however, for example, the following method is preferred. As the composition ratio of each element of the composite metal oxide catalyst, Re that is represented by the following formula is assumed. The denominator of Re represents the atomic ratio of molybdenum in a case in which it is assumed that each metal element and molybdenum are bonded at the ratio of 1 : 1 as an atomic ratio, the atomic ratio being the atomic ratio of residual molybdenum remaining as a result of bonding to a metal element other than bismuth. Thus, Re represents the atomic ratio of the residual molybdenum and bismuth in a composite metal oxide catalyst obtained by adding a metal element other than molybdenum and bismuth.

$$Re = (Content\ of\ Bi\ (mol))/\{(content\ of\ Mo\ (mol)) -$$

$$(content\ of\ metal\ components\ other\ than\ Mo\ and\ Bi\ (mol))\}$$

When the catalyst of the present invention is a supported catalyst, the content of the element that constitutes the support (Si, Al, or the like) is not included in this calculation.

For the catalyst of the present invention, it is preferable that Re is represented by the following Formula (C) :

$$0.02 \leq Re < 0.80 \qquad\qquad (C)$$

Re is preferably from 0.26 to 0.62, and more preferably from 0.37 to 0.51. In Non-Patent Literature 4, it is known that in regard to a catalyst composed only of molybdenum and bismuth, as the amount of bismuth is smaller, the percentage content of γ-bismuth molybdate decreases. Therefore, as in the case of the present invention, as the value of Re is lower in a composite metal oxide catalyst, the percentage content of γ-bismuth molybdate decreases as described above, and suppression of the production of the coke-like substance may be expected. However, in regard to a composite metal oxide catalyst to which a metal element other than molybdenum and bismuth has been added as in the case of the present invention, it has not been hitherto known specifically which range Re should be defined to be in, with industrially meaningful catalyst activity and yield being provided, in order to enable regulation of Rh and Ra, and it is also not known whether this is preferable for suppressing the production of the coke-like substance.

As another method of changing the ratio of the contents of γ-bismuth molybdate and α-bismuth molybdate, the calcination conditions may be utilized as disclosed in Non-Patent Literature 4. Preferred calcination conditions (calcination temperature, calcination time, time for temperature increase, and atmosphere during calcination) will be described below; however, since the calcination conditions affect the activity of the catalyst and the yield, selection of optimal conditions is definitely important along with suppression of the production of a coke-like substance. Selection of the calcination conditions for changing the ratio of the contents of γ-bismuth molybdate and α-bismuth molybdate is implemented regardless of, or in conjunction with, the regulation of Re by means of Formula (C) (regulation of the composition ratio of the composite metal oxide catalyst).

It is preferable that the catalyst of the present invention satisfies the composition formula represented by Formula (1):

$$Mo_{12}Bi_aFe_bCo_cNi_dX_eY_fZ_gO_h \qquad (1)$$

wherein Mo represents molybdenum; Bi represents bismuth; Fe represents iron; Co represents cobalt; Ni represents nickel; X represents at least one alkali metal element selected from lithium, sodium, potassium, rubidium, and cesium; Y represents at least one alkaline earth metal element selected from magnesium, calcium, strontium, and barium; Z represents at least one element selected from lanthanum, cerium, praseodymium, neodymium, samarium, europium, antimony, tungsten, lead, zinc, cerium, and thallium; O represents oxygen; a, b, c, d, e, and f represent the atomic ratios of bismuth, iron, cobalt, nickel, an alkali metal, an alkaline earth metal, and Z, respectively, with respect to molybdenum 12, in the ranges of $0.3 < a < 3.5$, $0.6 < b < 3.4$, $5 < c < 8$, $0 < d < 3$, $0 < e < 0.5$, $0 \leq f \ 4.0$, and $0 \leq g \leq 2.0$; and h represents a value satisfying the oxidation state of the other elements.

There are no particular limitations on the raw materials of each metal element for obtaining the catalyst of the present invention; however, nitrates, nitrites, sulfates, ammonium salts, organic acid salts, acetates, carbonates, subcarbonates, chlorides, inorganic acids, inorganic acid salts, heteropolyacids, heteropolyacid salts, hydroxides, oxides, metals, alloys, and the like, all of which include at least one of each metal element, and mixtures thereof may be used. Among these, preferred are nitrate raw materials.

The method for producing the catalyst of the present invention is not particularly limited; however, the production methods may be roughly classified into the following two types of production methods. For convenience, these methods will be referred to as Method (A) and Method (B) in the present invention. Method (A) is a method of obtaining the active components of the catalyst as a powder, and then molding this powder, and Method (B) is a method of bringing a solution in which the active components of the catalyst are dissolved, into contact with a support that has been molded in advance, and thereby supporting the active components on the support. The details of Method (A) and Method (B) will be described below.

A method for catalyst production according to Method (A) will be described below. The order of each process is described below as a preferred example; however, there are no particular limitations on the sequence of each process, the number of processes, and combinations of each process for obtaining the final manufactured product of catalyst.

Step (A1) Production and drying

A mixed solution or slurry of raw material compounds is prepared, and the mixed solution or slurry is subjected to the processes of a precipitation method, a gelation method, a co-precipitation method, a hydrothermal synthesis method, or the like. Subsequently, a dry powder of the present invention is obtained by using a known drying method such as a dry spraying method, an evaporation drying method, a drum drying method, or a freeze-drying method. Here, for the mixed solution or the slurry, the solvent may be any of water, an organic solvent, or a mixed solution thereof, and there are also no limitations on the raw material concentration of the active components of the catalyst. Furthermore, there are no particular limitations on the liquid temperature of the mixed solution or the slurry, the conditions for production such as the atmosphere, and the drying conditions; however, it is definitely adequate to select the conditions from appropriate ranges in consideration of the performance, mechanical strength, and moldability of the catalyst finally obtained, or the production efficiency. Among these, the most preferred method for the present invention is a method of forming a slurry of raw material of the active components of the catalyst in a temperature range of 20°C to 90°C, introducing this slurry into a spray dryer, and regulating the hot air inlet temperature, the pressure inside the spray dryer, and the flow amount of the slurry such that the dryer outlet temperature would be 70°C to 150°C, and the average particle size of the dry powder thus obtained would be 10 to 700 μm.

Step (A2) Preliminary calcination

The dry powder thus obtained is preliminarily calcined at 200°C to 600°C, and the preliminarily calcined powder of the present invention may be obtained. Here, also with regard to the conditions for the preliminary calcination, there are no particular limitations on the calcination time or the atmosphere during calcination, and the technique of calcination is also not particularly limited and may be selected from a fluidized bed, a rotary kiln, a muffle furnace, a tunnel calcination furnace, and the like. It is definitely adequate to select the conditions for the preliminary calcination from appropriate ranges in consideration of the performance, mechanical strength, and moldability of the catalyst finally obtained, or the production efficiency. Among these, the most preferred method for the present invention is a method of performing preliminary calcination in a tunnel calcination furnace at a temperature in the range of 300°C to 600°C for 1 to 12 hours in an air atmosphere.

Step (A3) Molding

The preliminarily calcined powder obtained as described above may be directly used as a catalyst, but may also be used after molding. The shape of the molded article is not particularly limited and may be selected from a spherical shape, a cylindrical shape, a ring shape, and the like. However, the shape should be selected in consideration of the mechanical strength of the catalyst that is finally obtainable after a series of production processes, the reactor, the production efficiency for the preparation, and the like. The molding method is also not particularly limited; however, when the support, molding aids, strength enhancing agent, binder, and the like that will be described in the following paragraph are added to the preliminarily calcined powder and molded into a cylindrical shape or a ring shape, a tablet molding machine, an extrusion molding machine or the like is used, or when the mixture is molded into a spherical shape, a granulator or the like is used to obtain a molded article. Among these, the most preferred method for the present invention is a method of coating an inert spherical support with the preliminarily calcined powder by a tumbling granulation method, and thus supporting and molding the catalyst.

Regarding the material for the support, known materials such as alumina, silica, titania, zirconia, niobia, silica-alumina, silicon carbide, a carbide, and mixtures thereof may be used. Furthermore, there are no particular limitations on the particle size, the water absorption rate and mechanical strength of the support, the degrees of crystallization of each crystal phase, or the mixing ratio of the crystal phases, and it is definitely adequate to select the conditions for the support from appropriate ranges in consideration of the performance and moldability of the catalyst finally obtained, or the production efficiency. The mixing ratio of the support and the preliminarily calcined powder is calculated as a support ratio by the following formula, based on the feed weights of each raw material.

$$\text{Support ratio (wt\%)} = \text{(Weight of the preliminarily calcined powder used for molding)} / \{\text{(weight of the preliminarily calcined powder used for molding)} + \text{(weight of the support used for molding)}\} \times 100$$

Regarding the raw materials other than the preliminarily calcined powder used for molding, the preliminarily calcined powder, as well as any types of a molding aid such as crystalline cellulose; a strength enhancing agent such as a ceramic whiskers; a binder such as an alcohol, a diol or a triol; and aqueous solutions thereof and the like may be used at any arbitrary mixing proportions to perform molding, and there are no particular limitations. It is also possible to introduce an element into the outermost surface of the catalyst in an embodiment that is different from Step (A1), by using a solution of the above-mentioned catalyst raw materials for this binder.

Step (A4) Main calcination

It is preferable that the preliminarily calcined powder or the molded article obtained as described above is calcined again (main calcination) at 200°C to 600°C before being used in a reaction. Also, in connection with the main calcination, there are no particular limitations on the calcination time or the atmosphere during calcination, the technique for calcination is also not particularly limited and may be selected from a fluidized bed, a rotary kiln, a muffle furnace, a tunnel calcination furnace, and the like. It is definitely adequate to select the conditions for the main calcination from appropriate ranges in consideration of the performance and mechanical strength of the catalyst finally obtained, or the production efficiency. Among these, the most preferred method for the present invention is a method of performing calcination in a tunnel calcination furnace at a temperature in the range of 300°C to 600°C for 1 to 12 hours in an air atmosphere.

Next, a method for catalyst production according to Method (B) will be described below. Each process will be sequentially described in the following description; however, there are no particular limitations on the sequence of each process, the number of processes, and combinations of each process for obtaining the final manufactured product of catalyst.

Step (B1) Impregnation

A solution or slurry into which the active components of the catalyst have been introduced is prepared, and a molded support or a catalyst obtained by Method (A) is impregnated with this solution or slurry. Thus, a molded article is obtained. Here, the method for supporting the active components of the catalyst by impregnation is not particularly limited and may be selected from a dipping method, an incipient wetness method, an ion exchanging method, a pH swing method, and the like. As the solvent for the solution or the slurry, any of water, an organic solvent, or a mixed solution thereof may be used, and there are also no limitations on the raw material concentrations of the active components of the catalyst. Furthermore, there are also no particular limitations on the liquid temperature of the mixed solution or the slurry, the pressure applied to the liquid, and the atmosphere around the liquid. However, it is definitely adequate to select the conditions from appropriate ranges in consideration of the performance, mechanical strength and moldability of the catalyst finally obtained, or the production efficiency. The shape of the molded support and the catalyst obtained by Method (A) are both not particularly limited and may be selected from a spherical shape, a cylindrical shape, a ring shape, a powdered form, and the like. There are also no particular limitations on the material, the particle size, the water absorption ratio, and the mechanical strength.

Step (B2) Drying

The molded article obtained as described above is subjected to a heat treatment at a temperature in the range of 20°C to 200°C using a known drying method such as an evaporation drying method, a drum drying method, or a freeze-drying method, and thus a catalyst dry molded product of the present invention is obtained. There are no particular limitations on the calcination time or the atmosphere during calcination, and the technique of calcination is also not particularly limited and may be selected from a fluidized bed, a rotary kiln, a muffle furnace, a tunnel calcination furnace, and the like. It is definitely adequate to select the conditions from appropriate ranges in consideration of the performance, mechanical strength and moldability of the catalyst finally obtained, or the production efficiency.

Step (B3) Main calcination

The catalyst dry molded product obtained as described above is subjected to a heat treatment at a temperature in the range of 200°C to 600°C using a known drying method such as an evaporation drying method, a drum drying method, or a freeze-drying method, and thus a catalyst of the present invention is obtained. Here, there are no particular limitations on the calcination time or the atmosphere during calcination, and the technique of calcination is also not particularly limited and may be selected from a fluidized bed, a rotary kiln, a muffle furnace, a tunnel calcination furnace, and the like. It is definitely adequate to select the conditions from appropriate ranges in consideration of the performance, mechanical strength and moldability of the catalyst finally obtained, or the production efficiency. Among these, the most preferred method for the present invention is a method of performing calcination in a tunnel calcination furnace at a temperature in the range of 300°C to 600°C for 1 to 12 hours in an air atmosphere.

The catalyst obtained by the production as described above is not particularly limited in view of the shape or the size; however, when the workability of packing into the reaction tube, the pressure loss in the reaction tube after packing, and the like are considered, the shape is preferably a spherical shape, the average particle size is preferably 3.0 to 10.0 mm, and the support ratio of the composite metal oxide catalyst is preferably 20% to 80% by weight.

It is preferable that the catalyst of the present invention has a certain mechanical strength at least before the initiation of the reaction, as will be described below. As described above, in a case in which a reaction for producing a conjugated diolefin from a monoolefin having 4 or more carbon atoms is carried out particularly in an industrial plant, the production of a coke-like substance and a regeneration treatment for combusting the coke-like substance are carried out in many occasions. Thus, if the catalyst has insufficient mechanical strength, damage of the catalyst caused by the production of a coke-like substance inside the catalyst, and damage of the catalyst and/or deterioration of the catalyst caused by the combustion gas in the regeneration treatment, may be considered. Also, there are concerns that the damaged catalyst accumulates within the reactor, and this may lead to various problems such as an increase in the pressure loss, undesirable reactions caused by the catalyst that locally accumulates within the reactor, and/or a decrease in yield, and incorporation into the purification system in the subsequent stages. Here, the mechanical strength of a catalyst is affected by various factors in the production process, such as the type or amount of each strength enhancing agent or binder added at the time of molding, or a combination thereof, the atomic ratio of the catalyst composition, the phase morphology of various crystal phases and proportions thereof, and the diameter, the geometric structure and the form of aggregation of secondary particles of the composite metal oxide catalyst formed in the production process or the drying process, and the mechanical strength is also closely related to the performance of the catalyst. That is, the object of the present invention more particularly relates to the demand for a catalyst which simultaneously satisfies suppression of the production of the coke-like substance, a high yield of the conjugated diolefin, and high mechanical strength in a reaction for producing a conjugated diolefin from a monoolefin having 4 or more carbon atoms.

The degree of wear, which is an index representing the mechanical strength according to the present invention, is calculated by the following method. A tester for the degree of wear of tablets manufactured by Hayashi Rikagaku K.K. is used as an apparatus, and 50 g of a catalyst sample is treated at a speed of rotation of 25 rpm for a treatment time of 10 minutes. Subsequently, the portion that has worn out is sieved with a standard sieve having a mesh size of 1.70 mm, and the weight of the catalyst remaining on the sieve is measured. The degree of wear is calculated by the following formula. As the value of the degree of wear is smaller, superior mechanical strength is obtained. A preferred range of the degree of wear is, for example, 3% by weigh tor less, more preferably 1.5% by weight or less, and even more preferably 0.5% by weight or less.

```
Degree of wear (wt%) = 100 × [(Weight of catalyst –

weight of catalyst remaining on the sieve)/weight of

catalyst]
```

The conditions for the reaction of producing a conjugated diolefin from a monoolefin having 4 or more carbon atoms by means of the catalyst of the present invention include the following: a mixed gas including 1% to 20% by volume of a monoolefin, 5% to 20% by volume of molecular oxygen, 0% to 60% by volume of water vapor, and 0% to 94% by volume of an inert gas, for example, nitrogen or carbon dioxide, is used, the reaction bath temperature is in the range of 200°C to 500°C, the reaction pressure is from normal pressure to an added pressure of 10 atmospheres, and the space velocity (GHSV) of the raw material gas with respect to the catalyst molded product of the present invention is in the range of 350 to 7,000 hr$^{-1}$. Regarding the form of the reaction, the reaction may be performed in a fixed bed, a movable bed, and a fluidized bed without any restrictions; however, a fixed bed is preferred.

The catalyst of the present invention may be used for a reaction by which a conjugated diolefin is produced from a mixed gas including a monoolefin having 4 or more carbon atoms and molecular oxygen by a catalytic oxidative dehydrogenation reaction. In a narrower sense, the catalyst of the present invention may be used for a reaction by which butadiene is produced from a mixed gas including n-butene and molecular oxygen by a catalytic oxidative dehydrogenation reaction.

n-Butene according to the present invention is intended to mean a single component gas selected from among 1-butene, trans-2-butene, cis-2-butene and isobutylene, or a mixed gas including at least one component, and more strictly, butadiene is intended to mean 1,3-butadiene.

The coke-like substance according to the present invention is produced from at least any one of the reaction raw materials, the reaction product, or the reaction byproducts in the reaction for producing a conjugated diolefin, and the details of the chemical composition or the mechanism of generation are not clearly understood. However, the coke-like substance is regarded as a causative substance that causes various problems, particularly such as inhibition of the circulation of reaction gases in an industrial plant, blocking of reaction tubes, and shutdown of the reaction resulting therefrom, when the coke-like substance is precipitated in or adheres to an inert substance, inside the reaction tube, or inside the facilities in the subsequent processes. Furthermore, for the purpose of avoiding the problems described above, in industrial plants, generally reactions are stopped before blocking occurs, and a regeneration treatment of combusting and removing the coke-like substance by raising the reaction bath temperature, or the like. Regarding the mechanism for the production of the coke-like substance, for example, the following is assumed. That is, examples may include, but not limited to, that at the time of using a composite metal oxide catalyst including molybdenum, the coke-like substance is caused by polymerization of various olefins and condensation of high boiling point compounds, starting from the molybdenum compounds that have sublimed and precipitated in the reactor; the coke-like substance is caused by polymerization of various olefins and condensation of high boiling point compounds, starting from the abnormal acid-base points and radical production points inside the catalyst and the reactor; and the coke-like substance is caused by production of high boiling point compounds as a result of the Diels-Alder reaction of the conjugated diene and other olefin compounds, and by condensation at sites where the temperature is locally low inside the reactor. In addition to these, various mechanisms are known.

According to the present invention, a catalyst that may suppress the production of a coke-like substance and may improve the long-term stability of the reaction, in regard to a reaction by which a conjugated diolefin is produced from a mixed gas including a monoolefin having 4 or more carbon atoms and molecular oxygen by catalytic oxidative dehydrogenation; and a method for producing the catalyst, are obtained.

Examples

Hereinafter, the present invention will be described in more detail by way of Examples; however, the present invention is not intended to be limited to the following Examples as long as the gist is maintained. In the following description, unless particularly stated otherwise, percentage (%) means mol%. Also, in the following description, the definitions of the n-butene conversion ratio, the butadiene yield, and TOS are as follows.

```
      n-Butene conversion ratio (mol%)

      = (Number of moles of reacted n-butene/number of moles of supplied n-butene) × 100

       Butadiene yield (mol%)

      = (Number of moles of produced butadiene/number of moles of supplied n-butene) × 100

        TOS = Mixed gas circulation time (hours)
```

X-ray diffraction peaks of the catalyst samples in Examples and Comparative Examples were measured as follows.
X-ray crystal structure analyzer: ULTIMA IV manufactured by Rigaku Co., Ltd.

Light source: Cu-K$\alpha$ radiation
Tube voltage: 40 kV
Tube current: 30 mA
Incidence slit and light reception slit: Open
Scan speed: 10°/min
Sampling width: 0.02°

In a case in which the catalyst sample was not a powder but a molded article, the X-ray peaks were analyzed after the sample was pulverized and mixed for 10 minutes with an agate mortar.

Example 1

(Production of Catalyst 1)

800 parts by weight of ammonium heptamolybdate was completely dissolved in 3,000 parts by weight of pure water that had been heated to 80°C (Mother Liquor 1). Next, 11 parts by weight of cesium nitrate was dissolved in 124 ml of pure water, and the solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the solution was added to Mother Liquor 1. Subsequently, 165 parts by weight of bismuth nitrate was dissolved in an aqueous solution of bismuth nitrate prepared by adding 42 parts by weight of nitric acid (60% by weight) to 175 ml of pure water that had been heated to 60°C, and the solution was added to Mother Liquor 1. This Mother Liquor 1 was dried by a spray drying method, and the dry powder thus obtained was preliminarily calcined under the conditions of 5 hours at 440°C. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 5 hours at 500°C, and Catalyst 1 of the present invention was obtained. The atomic ratio of Catalyst 1 calculated from the supplied raw materials was such that Mo : Bi : Fe : Co : Ni : Cs = 12 : 0.9 : 1.8 : 7.0 : 1.0 : 0.15, and the degree of wear was 0.41% by weight. The relative intensity ratios of the X-ray diffraction peaks of Catalyst 1 were such that Rh = 0.08, Ra = 0.10, and Re = 0.44.

Example 2

(Production of Catalyst 3)

Production was carried out in the same manner as in the case of Catalyst 1, except that the aqueous solution of bismuth nitrate added to Mother Liquor 1 in Example 1 was prepared as follows, and thus Catalyst 3 of the present invention was obtained. The aqueous solution of bismuth nitrate was prepared by adding 46 parts by weight of bismuth nitrate to an aqueous solution obtained by adding 12 parts by weight of nitric acid (60% by weight) to 49 ml of pure water that had been heated to 60°C. The atomic ratio of Catalyst 3 calculated from the supplied raw materials was such that Mo : Bi : Fe : Co : Ni : Cs = 12 : 0.3 : 1.8 : 7.0 : 1.0 : 0.15, and the relative intensity ratios of X-ray diffraction peaks were such that Rh = 0.44, Ra = 0.32, and Re = 0.12.

Example 3

(Production of Catalyst 4)

Production was carried out in the same manner as in the case of Catalyst 1, except that the aqueous solution of bismuth nitrate added to Mother Liquor 1 in Example 1 was prepared as follows, and thus Catalyst 4 of the present invention was obtained. The aqueous solution of bismuth nitrate was prepared by adding 83 parts by weight of bismuth nitrate to an aqueous solution obtained by adding 21 parts by weight of nitric acid (60% by weight) to 88 ml of pure water that had been heated to 60°C. The atomic ratio of Catalyst 4 calculated from the supplied raw materials was such that Mo : Bi : Fe : Co : Ni : Cs = 12 : 0.5 : 1.8 : 7.0 : 1.0 : 0.15, and the relative intensity ratios of X-ray diffraction peaks were such that Rh = 0.65, Ra = 0.35, and Re = 0.22.

Example 4

(Production of Catalyst 5)

Production was carried out in the same manner as in the case of Catalyst 1, except that the aqueous solution of bismuth nitrate added to Mother Liquor 1 in Example 1 was prepared as follows, and thus Catalyst 5 of the present invention was obtained. The aqueous solution of bismuth nitrate was prepared by adding 105 parts by weight of bismuth nitrate to an aqueous solution obtained by adding 27 parts by weight of nitric acid (60% by weight) to 112 ml of pure water that had been heated to 60°C. The atomic ratio of Catalyst 5 calculated from the supplied raw materials was such that Mo : Bi : Fe : Co : Ni : Cs = 12 : 0.6 : 1.8 : 7.0 : 1.0 : 0.15, and the relative intensity ratios of X-ray diffraction peaks were such that Rh = 0.58, Ra = 0.41, and Re = 0.28.

Example 5

(Production of Catalyst 6)

Production was carried out in the same manner as in the case of Catalyst 1, except that the aqueous solution of bismuth nitrate added to Mother Liquor 1 in Example 1 was prepared as follows, and thus Catalyst 6 of the present invention was obtained. The aqueous solution of bismuth nitrate was prepared by adding 120 parts by weight of bismuth nitrate to an aqueous solution obtained by adding 31 parts by weight of nitric acid (60% by weight) to 127 ml of pure water that had been heated to 60°C. The atomic ratio of Catalyst 6 calculated from the supplied raw materials was such that Mo : Bi : Fe : Co : Ni : Cs = 12 : 0.7 : 1.8 : 7.0 : 1.0 : 0.15, and the relative intensity ratios of X-ray diffraction peaks were such that Rh = 0.37, Ra = 0.41, and Re = 0.32.

Example 6

(Production of Catalyst 7)

Production was carried out in the same manner as in the case of Catalyst 1, except that the aqueous solution of bismuth nitrate added to Mother Liquor 1 in Example 1 was prepared as follows, and thus Catalyst 7 of the present invention was obtained. The aqueous solution of bismuth nitrate was prepared by adding 180 parts by weight of bismuth nitrate to an aqueous solution obtained by adding 46 parts by weight of nitric acid (60% by weight) to 191 ml of pure water that had been heated to 60°C. The atomic ratio of Catalyst 7 calculated from the supplied raw materials was such that Mo : Bi : Fe : Co : Ni : Cs = 12 : 1.0 : 1.8 : 7.0 : 1.0 : 0.15, and the relative intensity ratios of X-ray diffraction peaks were such that Rh = 0.46, Ra = 0.48, and Re = 0.48.

Example 7

(Production of Catalyst 8)

Production was carried out in the same manner as in the case of Catalyst 1, except that the aqueous solution of bismuth nitrate added to Mother Liquor 1 in Example 1 was prepared as follows, and thus Catalyst 8 of the present invention was obtained. The aqueous solution of bismuth nitrate was prepared by adding 195 parts by weight of bismuth nitrate to an aqueous solution obtained by adding 50 parts by weight of nitric acid (60% by weight) to 207 ml of pure water that had been heated to 60°C. The atomic ratio of Catalyst 8 calculated from the supplied raw materials was such that Mo : Bi : Fe : Co : Ni : Cs = 12 : 1.1 : 1.8 : 7.0 : 1.0 : 0.15, and the relative intensity ratios of X-ray diffraction peaks were such that Rh = 0.71, Ra = 0.33, and Re = 0.52.

Example 8

(Production of Catalyst 9)

Production was carried out in the same manner as in the case of Catalyst 1, except that the aqueous solution of bismuth nitrate added to Mother Liquor 1 in Example 1 was prepared as follows, and thus Catalyst 9 of the present invention was obtained. The aqueous solution of bismuth nitrate was prepared by adding 246 parts by weight of bismuth nitrate to an aqueous solution obtained by adding 63 parts by weight of nitric acid (60% by weight) to 261 ml of pure water that had been heated to 60°C. The atomic ratio of Catalyst 9 calculated from the supplied raw materials was such that Mo : Bi : Fe : Co : Ni : Cs = 12 : 1.3 : 1.8 : 7.0 : 1.0 : 0.15, and the relative intensity ratios of X-ray diffraction peaks were such that Rh = 0.57, Ra = 0.34, and Re = 0.66.

Example 9

(Production of Catalyst 10)

Production was carried out in the same manner as in the case of Catalyst 1, except that the aqueous solution of bismuth nitrate added to Mother Liquor 1 in Example 1 was prepared as follows, and thus Catalyst 10 of the present invention was obtained. The aqueous solution of bismuth nitrate was prepared by adding 302 parts by weight of bismuth nitrate to an aqueous solution obtained by adding 77 parts by weight of nitric acid (60% by weight) to 321 ml of pure water that had been heated to 60°C. The atomic ratio of Catalyst 10 calculated from the supplied raw materials was such that Mo : Bi : Fe : Co : Ni : Cs = 12 : 1.6 : 1.8 : 7.0 : 1.0 : 0.15, and the relative intensity ratios of X-ray diffraction peaks were such that Rh = 0.79, Ra = 0.50, and Re = 0.80.

Example 10

(Production of Catalyst 11)

Production was carried out in the same manner as in the case of Catalyst 1, except that the mixed aqueous solution of ferric nitrate, cobalt nitrate and nickel nitrate added to Mother Liquor 1 in Example 1 was prepared as follows, and thus Catalyst 11 of the present invention was obtained. The mixed aqueous solution was prepared by dissolving 275 parts by weight of ferric nitrate, 880 parts by weight of cobalt nitrate, and 110 parts by weight of nickel nitrate in 666 ml of pure water that had been heated to 60°C. The atomic ratio of Catalyst 11 calculated from the supplied raw materials was such that Mo : Bi : Fe : Co : Ni : Cs = 12 : 0.9 : 1.8 : 8.3 : 1.0 : 0.15, and the relative intensity ratios of X-ray diffraction peaks were such that Rh = 0.73, Ra = 0.43, and Re = 0.80.

Example 11

(Production of Catalyst 12)

Production was carried out in the same manner as in the case of Catalyst 1, except that the mixed aqueous solution of ferric nitrate, cobalt nitrate and nickel nitrate added to Mother Liquor 1 in Example 1 was prepared as follows, and thus Catalyst 12 of the present invention was obtained. The mixed aqueous solution was prepared by dissolving 275 parts by weight of ferric nitrate, 769 parts by weight of cobalt nitrate, and 210 parts by weight of nickel nitrate in 665 ml of pure water that had been heated to 60°C. The atomic ratio of Catalyst 12 calculated from the supplied raw materials was such that Mo : Bi : Fe : Co : Ni : Cs = 12 : 0.9 : 1.8 : 7.0 : 1.9 : 0.15, and the relative intensity ratios of X-ray diffraction peaks were such that Rh = 0.22, Ra = 0.17, and Re = 0.79.

Example 12

(Production of Catalyst 13)

Production was carried out in the same manner as in the case of Catalyst 1, except that the aqueous solution of cesium nitrate added to Mother Liquor 1 in Example 1 was prepared as follows, and thus Catalyst 13 of the present invention was obtained. The aqueous solution of cesium nitrate was prepared by dissolving 3.5 parts by weight of bismuth nitrate in 40 ml of pure water. The atomic ratio of Catalyst 13 calculated from the supplied raw materials was such that Mo : Bi : Fe : Co : Ni : Cs = 12 : 0.9 : 1.8 : 7.0 : 1.0 : 0.05, and the relative intensity ratios of X-ray diffraction peaks were such that Rh = 0.34, Ra = 0.34, and Re = 0.42.

Example 13

(Production of Catalyst 14)

Production was carried out in the same manner as in the case of Catalyst 1, except that after the aqueous solution of bismuth nitrate was added to Mother Liquor 1 in Example 1, the following aqueous solution was prepared and added to Mother Liquor 1. Thus, Catalyst 14 of the present invention was obtained. The aqueous solution was prepared by dissolving 80 parts by weight of calcium nitrate in 88 ml of pure water. The atomic ratio of Catalyst 14 calculated from the supplied raw materials was such that Mo : Bi : Fe : Co : Ni : Cs : Ca = 12 : 0.9 : 1.8 : 7.0 : 1.0 : 0.15 : 0.9, and the relative intensity ratios of X-ray diffraction peaks were such that Rh = 0.76, Ra = 0.49, and Re = 0.78.

Example 14

(Production of Catalyst 15)

Production was carried out in the same manner as in the case of Catalyst 1, except that after the aqueous solution of bismuth nitrate was added to Mother Liquor 1 in Example 1, the following aqueous solution was prepared and added to Mother Liquor 1. Thus, Catalyst 15 of the present invention was obtained. The aqueous solution was prepared by dissolving 87 parts by weight of magnesium nitrate in 96 ml of pure water. The atomic ratio of Catalyst 15 calculated from the supplied raw materials was such that Mo : Bi : Fe : Co : Ni : Cs : Mg = 12 : 0.9 : 1.8 : 7.0 : 1.0 : 0.15 : 0.9, and the relative intensity ratios of X-ray diffraction peaks were such that Rh = 0.76, Ra = 0.48, and Re = 0.78.

Example 15

(Production of Catalyst 16)

Production was carried out in the same manner as in the case of Catalyst 1, except that the aqueous solution of bismuth nitrate added to Mother Liquor 1 in Example 1 was prepared as follows, and thus Catalyst 16 of the present invention was obtained. The aqueous solution of bismuth nitrate was prepared by adding 4.0 parts by weight of bismuth nitrate to an aqueous solution obtained by adding 1.0 parts by weight of nitric acid (60% by weight) to 4.2 ml of pure water that had been heated to 60°C. The atomic ratio of Catalyst 16 calculated from the supplied raw materials was such that Mo : Bi : Fe : Co : Ni : Cs = 12 : 0.02 : 1.8 : 7.0 : 1.0 : 0.15, and the relative intensity ratios of X-ray diffraction peaks were such that Rh = 0.07, Ra = 0.07, and Re = 0.01.

Example 16

(Production of Catalyst 17)

Production was carried out in the same manner as in the case of Catalyst 1, except that the aqueous solution of bismuth nitrate added to Mother Liquor 1 in Example 1 was prepared as follows, and thus Catalyst 17 of the present invention was obtained. The aqueous solution of bismuth nitrate was prepared by adding 10 parts by weight of bismuth nitrate to an aqueous solution obtained by adding 2.5 parts by weight of nitric acid (60% by weight) to 11 ml of pure water that had been heated to 60°C. The atomic ratio of Catalyst 17 calculated from the supplied raw materials was such that Mo : Bi : Fe : Co : Ni : Cs = 12 : 0.1 : 1.8 : 7.0 : 1.0 : 0.15, and the relative intensity ratios of X-ray diffraction peaks were such that Rh = 0.10, Ra = 0.11, and Re = 0.03.

Comparative Example 1

(Production of Catalyst 2)

800 parts by weight of ammonium heptamolybdate was completely dissolved in 3,000 parts by weight of pure water that had been heated to 80°C (Mother Liquor 1). Next, 11 parts by weight of cesium nitrate was dissolved in 124 ml of pure water, and the solution was added to Mother Liquor 1. Next, 275 parts by weight of ferric nitrate, 769 parts by weight of cobalt nitrate, and 110 parts by weight of nickel nitrate were dissolved in 612 ml of pure water that had been heated to 60°C, and the solution was added to Mother Liquor 1. Subsequently, 311 parts by weight of bismuth nitrate was dissolved in an aqueous solution of bismuth nitrate prepared by adding 79 parts by weight of nitric acid (60% by weight) to 330 ml of pure water that had been heated to 60°C, and the solution was added to Mother Liquor 1. This Mother Liquor 1 was dried by a spray drying method, and the dry powder thus obtained was preliminarily calcined under the conditions of 5 hours at 440°C. A portion equivalent to 5% by weight of crystalline cellulose was added to the preliminarily calcined powder thus obtained, the mixture was sufficiently mixed, and then the mixture was supported on an inert spherical support such that the support ratio would be 50% by weight, and was molded into a spherical shape, by a tumbling granulation method using a 33 wt% glycerin solution as a binder. The spherical molded article thus obtained was calcined under the conditions of 5 hours at 520°C, and Catalyst 2 for comparison was obtained. The atomic ratio of Catalyst 2 calculated from the supplied raw materials was such that Mo : Bi : Fe : Co : Ni : Cs = 12 : 1.7 : 1.8 : 7.0 : 1.0 : 0.15, and the degree of wear was 0.31% by weight. The relative intensity ratios of the X-ray diffraction peaks of Catalyst 2 were such that Rh = 0.93, Ra = 0.56, and Re = 0.83.

Test Example 1

(Coke precipitation reaction)

Catalyst 1 obtained in Example 1 was reacted and evaluated by the following method. 53 ml of the catalyst was packed in a stainless steel reaction tube, and the catalyst was caused to react for a TOS of 290 hours using a mixed gas having a gas volume ratio of 1-butene : oxygen : nitrogen : water vapor = 1 : 1 : 7 : 1, under the conditions of normal pressure and a GHSV of 1,200 hr$^{-1}$, by changing the reaction bath temperature so as to maintain the 1-butene conversion ratio = 98.0 $\pm$ 1.0%. Thus, a coke-like substance was precipitated on the catalyst. A liquid component and a gas component were separated at the reaction tube outlet using a condenser, and each component in the gas component was quantitatively analyzed using a gas chromatograph equipped with a hydrogen flame ionization detector and a thermal conductivity detector. Each data obtained by gas chromatography was compensated by multiplying by a factor, and thus the 1-butene conversion ratio and the butadiene yield were calculated. The butadiene yield for the TOS of 260 hours was 86.2%.

(Coke combustion reaction)

After the coke precipitation reaction, for the purpose of combusting the coke-like substance precipitated on the catalyst, a combustion reaction was performed for about a TOS of 10 hours at a reaction bath temperature of 400°C using a mixed gas having a gas volume ratio of oxygen : nitrogen = 1 : 3 at normal pressure and at a space velocity of 400 hr$^{-1}$. A quantitative analysis similar to that performed for the coke precipitation reaction was performed, and the amount of the coke-like substance was calculated from the cumulative amounts of production of $CO_2$ and CO in the reaction tube outlet gas, as a percentage with respect to the weight of the packed catalyst.

Test Example 2

The amount of the coke-like substance precipitated under the same reaction conditions as in Test Example 1, except that the TOS of the coke precipitation reaction was changed to 360 hours, was evaluated. The butadiene yield for a TOS of 330 hours was 86.2%.

Test Example 3

The amount of the coke-like substance precipitated under the same reaction conditions as in Test Example 1, except that the catalyst to be evaluated was changed to Catalyst 3 obtained in Example 2, was evaluated.

Test Example 4

The amount of the coke-like substance precipitated under the same reaction conditions as in Test Example 1, except that the catalyst to be evaluated was changed to Catalyst 4 obtained in Example 3, was evaluated.

Test Example 5

The amount of the coke-like substance precipitated under the same reaction conditions as in Test Example 1, except that the catalyst to be evaluated was changed to Catalyst 5 obtained in Example 4, was evaluated.

Test Example 6

The amount of the coke-like substance precipitated under the same reaction conditions as in Test Example 1, except that the catalyst to be evaluated was changed to Catalyst 6 obtained in Example 5, was evaluated.

Test Example 7

The amount of the coke-like substance precipitated under the same reaction conditions as in Test Example 1, except that the catalyst to be evaluated was changed to Catalyst 7 obtained in Example 6, was evaluated.

Test Example 8

The amount of the coke-like substance precipitated under the same reaction conditions as in Test Example 1, except that the catalyst to be evaluated was changed to Catalyst 8 obtained in Example 7, was evaluated.

Test Example 9

The amount of the coke-like substance precipitated under the same reaction conditions as in Test Example 1, except that the catalyst to be evaluated was changed to Catalyst 9 obtained in Example 8, was evaluated.

Test Example 10

The amount of the coke-like substance precipitated under the same reaction conditions as in Test Example 1, except that the catalyst to be evaluated was changed to Catalyst 10 obtained in Example 9, was evaluated.

Test Example 11

The amount of the coke-like substance precipitated under the same reaction conditions as in Test Example 1, except that the catalyst to be evaluated was changed to Catalyst 11 obtained in Example 10, was evaluated.

Test Example 12

The amount of the coke-like substance precipitated under the same reaction conditions as in Test Example 1, except that the catalyst to be evaluated was changed to Catalyst 12 obtained in Example 11, was evaluated.

Test Example 13

The amount of the coke-like substance precipitated under the same reaction conditions as in Test Example 1, except that the catalyst to be evaluated was changed to Catalyst 13 obtained in Example 12, was evaluated.

Test Example 14

The amount of the coke-like substance precipitated under the same reaction conditions as in Test Example 1, except that the catalyst to be evaluated was changed to Catalyst 14 obtained in Example 13, was evaluated.

Test Example 15

The amount of the coke-like substance precipitated under the same reaction conditions as in Test Example 1, except that the catalyst to be evaluated was changed to Catalyst 15 obtained in Example 14, was evaluated.

Test Example 16

The amount of the coke-like substance precipitated under the same reaction conditions as in Test Example 1, except that the catalyst to be evaluated was changed to Catalyst 16 obtained in Example 15, was evaluated.

Test Example 17

The amount of the coke-like substance precipitated under the same reaction conditions as in Test Example 1, except that the catalyst to be evaluated was changed to Catalyst 17 obtained in Example 16, was evaluated.

Comparative Test Example 1

The amount of the coke-like substance precipitated under the same reaction conditions as in Test Example 1, except that the catalyst to be evaluated was changed to Catalyst 2 obtained in Comparative Example 1, was evaluated. The butadiene yield for a TOS of 260 hours was 87.5%.

Comparative Test Example 2

The amount of the coke-like substance precipitated under the same reaction conditions as in Comparative Test Example 1, except that the TOS of the coke precipitation reaction was changed to 360 hours, was evaluated. The butadiene yield for a TOS of 330 hours was 85.5%.
The results for the coke precipitation reactions performed in Test Examples and Comparative Test Examples described above are presented in Table 1. As is obvious from Table 1, it is understood that in regard to the catalysts of the present invention, if the relative intensity ratios of X-ray diffraction peaks are within the ranges defined in the present invention, the production of the coke-like substance can be suppressed to a large extent.

[Table 1]

| | Catalyst | Rh | Ra | Re | Coke precipitation reaction TOS [hr] | Amount of coke-like substance [wt%] |
|---|---|---|---|---|---|---|
| Test Example 1 | Catalyst 1 | 0.08 | 0.10 | 0.44 | 290 | 0.010 |
| Test Example 2 | Catalyst 1 | 0.08 | 0.10 | 0.44 | 360 | 0.35 |
| Test Example 3 | Catalyst 3 | 0.44 | 0.32 | 0.12 | 290 | 0.007 |
| Test Example 4 | Catalyst 4 | 0.65 | 0.35 | 0.22 | 290 | 0.008 |
| Test Example 5 | Catalyst 5 | 0.58 | 0.41 | 0.28 | 290 | 0.004 |
| Test Example 6 | Catalyst 6 | 0.37 | 0.41 | 0.32 | 290 | 0.007 |
| Test Example 7 | Catalyst 7 | 0.46 | 0.48 | 0.48 | 290 | 0.006 |
| Test Example 8 | Catalyst 8 | 0.71 | 0.33 | 0.52 | 290 | 0.006 |
| Test Example 9 | Catalyst 9 | 0.57 | 0.34 | 0.66 | 290 | 0.009 |
| Test Example 10 | Catalyst 10 | 0.79 | 0.50 | 0.80 | 290 | 0.028 |
| Test Example 11 | Catalyst 11 | 0.73 | 0.3 | 0.80 | 290 | 0.013 |
| Test Example 12 | Catalyst 12 | 0.22 | 0.17 | 0.79 | 290 | 0.013 |

(continued)

|  | Catalyst | Rh | Ra | Re | Coke precipitation reaction TOS [hr] | Amount of coke-like substance [wt%] |
|---|---|---|---|---|---|---|
| Test Example 13 | Catalyst 13 | 0.34 | 0.34 | 0.42 | 290 | 0.004 |
| Test Example 14 | Catalyst 14 | 0.76 | 0.49 | 0.78 | 290 | 0.022 |
| Test Example 15 | Catalyst 15 | 0.76 | 0.48 | 0.78 | 290 | 0.021 |
| Test Example 16 | Catalyst 16 | 0.07 | 0.07 | 0.01 | 290 | 0.093 |
| Test Example 17 | Catalyst 17 | 0.10 | 0.11 | 0.03 | 290 | 0.073 |
| Comparative Test Example 1 | Catalyst 2 | 0.93 | 0.56 | 0.83 | 290 | 0.60 |
| Comparative Test Example 2 | Catalyst 2 | 0.93 | 0.56 | 0.83 | 360 | 1.89 |

**Claims**

1. A composite metal oxide catalyst for producing a conjugated diolefin from a mixed gas including a monoolefin having 4 or more carbon atoms and molecular oxygen by a catalytic oxidative dehydrogenation reaction, the composite metal oxide catalyst having a relative intensity range of X-ray diffraction peaks represented by the following Formula (A):

$$0 \leq Rh \ (= Ph1/Ph2) < 0.8 \hspace{3cm} (A)$$

wherein Ph1 represents the maximum peak height within the range of $2\theta = 28.1° \pm 0.2°$ for the X-ray diffraction peaks; Ph2 represents the maximum peak height within the range of $2\theta = 27.9° \pm 0.2°$ for the X-ray diffraction peaks; and Rh represents the relative intensity ratio of Ph1 with respect to Ph2.

2. A composite metal oxide catalyst for producing a conjugated diolefin from a mixed gas including a monoolefin having 4 or more carbon atoms and molecular oxygen by a catalytic oxidative dehydrogenation reaction, the composite metal oxide catalyst having a relative area intensity range of X-ray diffraction peaks represented by the following Formula (B):

$$0 \leq Ra \ (= Pa1/Pa2) < 0.5 \hspace{3cm} (B)$$

wherein Pa1 represents the peak area obtainable by fitting within the range of $2\theta = 28.1° \pm 0.2°$ for the X-ray diffraction peaks; Pa2 represents the peak area obtainable by fitting within the range of $2\theta = 27.9°$ for the X-ray diffraction peaks; and Ra represents the relative area intensity ratio of Pa1 with respect to Pa2.

3. The composite metal oxide catalyst according to claim 1 or claim 2, wherein the composite metal oxide catalyst comprises molybdenum (Mo), bismuth (Bi), an alkali metal (AM), and if necessary, an alkaline earth metal (AEM), and the molar ratio of each component satisfies the following Formula (C):

$$0.02 \leq Re < 0.80 \hspace{3cm} (C)$$

wherein Re = (Content (mol) of Bi)/{(content (mol) of Mo) - (content (mol) of metal components other than Mo and Bi)}.

**4.** The composite metal oxide catalyst according to any one of claims 1 to 3, wherein the composite metal oxide catalyst satisfies the following composition formula:

$$Mo_{12}Bi_aFe_bCo_cNi_dX_eY_fZ_gO_h$$

wherein Mo represents molybdenum; Bi represents bismuth; Fe represents iron; Co represents cobalt; Ni represents nickel; X represents at least one alkali metal element selected from lithium, sodium, potassium, rubidium, and cesium; Y represents at least one alkaline earth metal element selected from magnesium, calcium, strontium, and barium; Z represents at least one element selected from lanthanum, cerium, praseodymium, neodymium, samarium, europium, antimony, tungsten, lead, zinc, cerium, and thallium; O represents oxygen; a, b, c, d, e, and f represent the atomic ratios of bismuth, iron, cobalt, nickel, the alkali metal, the alkaline earth metal, and Z, respectively, with respect to molybdenum 12, and in the ranges of $0.3 < a < 3.5$, $0.6 < b < 3.4$, $5 < c < 8$, $0 < d < 3$, $0 < e < 0.5$, $0 \leq f \leq 4.0$, and $0 \leq g \leq 2.0$; and h represents a value satisfying the oxidation states of other elements.

**5.** A method for producing the composite metal oxide catalyst for conjugated diolefin production according to claim 3, the method comprising:

a step of preparing a slurry including a compound containing molybdenum; a compound containing bismuth; a compound containing at least one alkali metal; and if necessary, a compound containing at least one alkaline earth metal, and drying the slurry to obtain a dry powder; and
a step of calcining the dry powder at a temperature of from 200°C to 600°C.

**6.** A method for producing the composite metal oxide catalyst for conjugated diolefin production according to claim 4, the method comprising:

a step of preparing a slurry including a compound containing molybdenum; a compound containing bismuth; a compound containing iron; a compound containing cobalt; a compound containing nickel; a compound containing at least one alkali metal element selected from lithium, sodium, potassium, rubidium, and cesium; a compound containing at least one alkaline earth metal element selected from magnesium, calcium, strontium, and barium; and a compound containing at least one element Z selected from lanthanum, cerium, praseodymium, neodymium, samarium, europium, antimony, tungsten, lead, zinc, cerium, and thallium,
provided that a, b, c, d, e, and f represent the atomic ratios of bismuth, iron, cobalt, nickel, the alkali metal, the alkaline earth metal, and Z, respectively, with respect to molybdenum 12, and in the ranges of $0.3 < a < 3.5$, $0.6 < b < 3.4$, $5 < c < 8$, $0 < d < 3$, $0 < e < 0.5$, $0 \leq f \leq 4.0$, $0 \leq g \leq 2.0$;
a step of drying the slurry to obtain a dry powder; and
a step of calcining the dry powder at a temperature of from 200°C to 600°C.

**7.** A supported catalyst for conjugated diolefin production, the supported catalyst comprising the composite metal oxide catalyst according to any one of claims 1 to 4 supported on a support.

**8.** The supported catalyst according to claim 7, wherein the support is a spherical support.

**9.** The supported catalyst according to claim 7 or 8, wherein the average particle size is from 3.0 mm to 10.0 mm, and the support ratio of the composite metal oxide catalyst is from 20% by weight to 80% by weight.

**10.** A method for producing the supported catalyst for conjugated diolefin production according to any one of claims 7 to 9, the method comprising a molding step of coating the support with the composite metal oxide catalyst together with a binder.

**EP 3 266 521 A1**

<table>
<tr><td colspan="3"><b>INTERNATIONAL SEARCH REPORT</b></td><td>International application No.<br>PCT/JP2016/056416</td></tr>
</table>

A. CLASSIFICATION OF SUBJECT MATTER
*B01J23/887*(2006.01)i, *B01J35/08*(2006.01)i, *B01J37/02*(2006.01)i, *B01J37/08*(2006.01)i, *C07C5/48*(2006.01)i, *C07C11/167*(2006.01)i, *C07B61/00*(2006.01)n

According to International Patent Classification (IPC) or to both national classification and IPC

B. FIELDS SEARCHED

Minimum documentation searched (classification system followed by classification symbols)
B01J23/887, B01J35/08, B01J37/02, B01J37/08, C07C5/48, C07C11/167, C07B61/00

Documentation searched other than minimum documentation to the extent that such documents are included in the fields searched
Jitsuyo Shinan Koho        1922–1996    Jitsuyo Shinan Toroku Koho    1996–2016
Kokai Jitsuyo Shinan Koho  1971–2016    Toroku Jitsuyo Shinan Koho    1994–2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2013-202459 A (Mitsubishi Chemical Corp.), 07 October 2013 (07.10.2013), claims 1 to 4; paragraphs [0051] to [0062]; fig. 2 (Family: none) | 1–10 |
| A | JP 2013-146655 A (Mitsubishi Chemical Corp.), 01 August 2013 (01.08.2013), claims 1 to 5; paragraphs [0053] to [0069] (Family: none) | 1–10 |
| A | JP 60-149534 A (Nippon Shokubai Kagaku Kogyo Co., Ltd.), 07 August 1985 (07.08.1985), claims (Family: none) | 1–10 |

☐ Further documents are listed in the continuation of Box C.    ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 March 2016 (24.03.16) | 05 April 2016 (05.04.16) |

| Name and mailing address of the ISA/<br>Japan Patent Office<br>3-4-3,Kasumigaseki,Chiyoda-ku,<br>Tokyo 100-8915,Japan | Authorized officer<br><br>Telephone No. |
|---|---|

Form PCT/ISA/210 (second sheet) (January 2015)

20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013100244 A
- JP 2011148765 A
- JP 2013146655 A
- JP 2013202459 A

**Non-patent literature cited in the description**

- **M.E. LEVIN et al.** *J. Haz. Mat.,* 2004, vol. 115, 71-90
- **W. MARTIR et al.** *J. Am. Chem. Soc.,* 1981, vol. 103, 3728-3732
- **J.H. LUNSFORD.** *Langmuir,* 1989, vol. 5, 12-16
- **M.T. LE et al.** *App. Cat. A,* 2005, vol. 282, 189-194